# EUROPEAN PATENT APPLICATION

(11) **EP 3 306 315 A1**
(43) Date of publication of application: **11.04.2018**
(21) Application number: 17194998.5
(22) Date of filing: 05.10.2017
(51) Int. Cl.: G01N 33/49

(54) **METHOD FOR COLLECTING RARE CELLS**

(30) Priority: 06.10.2016 JP 2016198422; 04.10.2017 JP 2017194023
(71) Applicant: ARKRAY, Inc., Minami-ku Kyoto-shi, Kyoto 601-8045 (JP)
(72) Inventor: TAKAGI, Hidenori, Kyoto-shi, Kyoto 602-0008 (JP); KOZUKA, Masahiro, Kyoto-shi, Kyoto 602-0008 (JP); ITO, Hiroshi, Kyoto-shi, Kyoto 602-0008 (JP)
(74) Representative: Jones, Elizabeth Louise

(57) **Abstract**

Provided is a method by which target cells can be collected from a specimen containing target cells and non-target cells at a high purity. The present disclosure relates to a method for collecting rare cells including: a step of filtering a specimen containing target rare cells and non-target cells with a filter capable of capturing the target rare cells; a step of labeling the non-target cells among the r cells that are captured by the filter at the filter; a step of separating the labeled non-target cells from a solution containing the rare cells and the labeled non-target cells, which collected from the filter, and to collect the solution containing the rare cells; and a step of capturing the rare cells from the solution containing the rare cells.

## Description

### BACKGROUND OF THE INVENTION

### 1. Field of the Invention

The present disclosure relates to a method for collecting or detecting cells, and a cell collection system. The present disclosure specifically relates to a method for collecting or detecting rare cells in blood, and a rare cell collection system used therefor.

### 2. Description of Related Art

Blood contains various components such as red blood cells, white blood cells, and platelets. For the purposes of early detection and diagnosis of diseases, these components included in blood are examined.

Blood contains rare cells such as circulating tumor cells (CTCs) and immune cells, and these cells are medically important cells. CTCs are cancer cells (tumor cells) that separate from primary tumor tissues or metastatic tumor tissues and circulate through blood flow. CTCs are said to metastasize by being carried to the other organs or the like due to CTCs circulating through this blood flow. Thus, CTCs in blood are recognized as useful as a factor for determining the effect of treating a metastatic cancer or predicting prognosis of a metastatic cancer, and thus CTCs have been analyzed actively.

WO 2011/108454 discloses a method for letting a filter capture rare cells (CTCs) in blood by treating blood with the filter, and assaying the captured rare cells. PLOS ONE, April 2013, Volume 8, Issue 4, e61770 discloses a method in which a cell suspension containing white blood cells and rare cells is prepared, all of the cells included in this cell suspension are spread in an observation region provided with a plurality of holes, and rare cells are detected in an image obtained through optical imaging.

### SUMMARY OF THE INVENTION

However, with the above-described method, non-target cells (for example, white blood cells) are separated insufficiently and a large amount of non-target cells remain, and thus this method is problematic in that assay of target cells (for example, rare cells) needs a lot of time and effort. Also, there is a problem that results of assays of target cells cannot be accurately obtained due to noise derived from non-target cells.

Furthermore, there is a problem in that the concentration of rare cells in a specimen is significantly low when rare cells are assayed, and therefore several milliliters of the specimen need to be treated, but in order to handle a small amount of cells in the subsequent assay, it is necessary to handle cells in a small amount. Also, there is a problem in that the results of assays of target cells cannot be accurately obtained due to a low concentration of rare cells in the specimen.

There is a problem in that in order to analyze genes of rare cells, it is necessary to collect cells as a highly pure sample with a small amount in a state in which the sample does not inhibit genetic analysis.

In one or more embodiments, the present disclosure provides a method by which target cells can be collected with a high purity from a specimen containing target cells and non-target cells, and a system used therefor.

In one aspect, the present disclosure relates to a method for collecting rare cells in a specimen, the method including:
filtering step: a step of filtering a specimen containing target rare cells and non-target cells with a filter capable of capturing the target rare cells;
labeling step: a step of labeling the non-target cells among the cells that are captured by the filter, at the filter;
separation/collection step: a step of separating the labeled non-target cells from a solution comprising the rare cells and the labeled non-target cells, which collected from the filter, and collecting the solution containing the rare cells; and
capture step: a step of capturing the rare cells from the solution containing the rare cells.

According to the present disclosure, in one or more embodiments, it is possible to provide a method by which target cells can be collected from a specimen containing target rare cells and non-target cells, with a high purity and a small amount of liquid. That is, according to the present disclosure, it is possible to obtain target cells in purity and liquid amount levels that are sufficient for genetic testing. Also, according to the present disclosure, in one or more embodiments, the above-described effects can be obtained with only a minimum of four steps (e.g., the filtering step, the labeling step, the separation/collection step, the capture step, and the like) without performing a nucleic acid purification step.

### BRIEF DESCRIPTION OF THE DRAWINGS

FIG. 1 is a flowchart showing one example of an operation example in one embodiment of a collection method of the present disclosure.
FIG. 2 is a schematic diagram of a configuration of a cell capture/staining apparatus used in the Example.
FIG. 3 is a schematic diagram of a configuration of a magnetic separation apparatus used in the Example.
FIG. 4 is a schematic diagram of a configuration of a dielectrophoretic apparatus used in the Example.
FIG. 5 is an image showing one example of the results of the Example (the results of detection of rare cells and white blood cells).
FIG. 6 is a graph showing one example of the results of the Example (the results of detection of an EGFR genetic mutation).

### DETAILED DESCRIPTION OF THE INVENTION

In order to accurately assay target cells in a specimen, target cells need to be collected from a specimen with a high purity. In a case where common cells are target cells, the specimen contains a relatively large amount of target cells, and thus the purity of target cells can be increased by repeating a purification step. On the other hand, in a case where rare cells, such as CTCs, are target cells, a specimen such as blood contains an extremely small amount of rare cells (for example, 8 mL of blood contains about several cells (e.g. about 0 to 8 cells)) and a large amount of non-target cells (for example, white blood cells and the like) in addition to the rare cells. Thus, when the purification step is repeated, even though non-target cells can be removed, there is a risk that rare cells will be lost. Therefore, not only purity but also a reduction in loss of target rare cells are required to collect rare cells. Also, rare cells are assayed through observation with a microscope or the like, genetic analysis such as PCR, and the like, and thus the amount of liquid collected after the purification step needs be small.

In one aspect, the present disclosure is based on the finding that target rare cells (hereinafter also referred to as target cells) with a high purity can be collected from a specimen containing target cells and non-target cells (for example, white blood cells) included in a larger amount than the target cells, by performing at least three steps as a series of flows: a purification step and concentration step (for example, the filtering step with a filter), an additional purification and additional concentration step (for example, the separation/collection step and the capture step), and a step of, during any step among all of the steps (preferably, after the filtering step), labeling cells for the purification or concentration step that will be performed after said step.

The collection method of the present disclosure may include a pair of steps, such as "the purification step and the concentration step" at least two times, and include other treating steps, and the above-described "all of the steps" means all of the steps performed in this collection method, and means performing the above-described labeling treatment during any step among all of the steps. That is, the above-described labeling treatment may be implemented during the purification step and the concentration step, or during a step other than the purification step and concentration step.

In one or more embodiments, the purification step and concentration step may use a method in which a difference in physical properties between target rare cells and non-target cells is utilized, and examples thereof include physical separation with a filter and separation with a density gradient. In one or more embodiments, the additional purification step and the additional concentration step may use a method in which a difference in biological properties between target rare cells and non-target cells is utilized and/or a method in which a difference in physical properties between target rare cells and non-target cells is utilized, and examples thereof include magnetic separation, suction, flow cytometry, and dielectrophoresis. In one or more embodiments, examples of the additional concentration step include dielectrophoresis, magnetic separation, suction, and flow cytometry. However, the present disclosure is not limited to the above-described examples.

In one aspect, the present disclosure is based on new findings found by the inventors that target cells were collected as described above from a specimen containing target cells and a larger amount of non-target cells (for example, white blood cells) than the target cells, and the target cells were collected at a high purity through the labeling treatment by labeling, at the filter, non-target cells among cells that were captured by the filter.

In one aspect, the present disclosure is based on a new finding found by the inventors that a specimen containing target rare cells and non-target cells was filtered using a filter capable of capturing rare cells, non-target cells were labeled at the filter by which the rare cells and non-target cells were captured, the labeled non-target cells and the rare cells were collected, a solution containing the rare cells was collected by selectively separating the non-target cells and the rare cells using this label, and the rare cells in that solution were captured, and thereby the small amount of rare cells included in the specimen were collected at a high purity and a high remaining ratio while significantly reducing loss during treatments. In the present disclosure, "labeling on a filter" refers to labeling non-target cells without collecting cells captured through filtering with the above-described filter from this filter (in a state in which rare cells are substantially captured by the filter).

The mechanism with which target cells can be collected from the specimen with a high purity by the method of the present disclosure is not clear, but it is inferred as follows.

The efficiency of separation of target cells and non-target cells can be increased by performing at least two stages of purification steps using different principles, namely, the purification step and concentration step in which purification is performed by removing a portion of the non-target cells in the specimen utilizing a difference in physical properties between target cells and non-target cells, such as filtering with a filter, and the additional purification step and concentration step performed using at least one of the method in which a difference in biological properties between target rare cells and non-target cells is utilized, such as magnetic separation and dielectrophoresis, and/or the method in which a difference in physical properties between target rare cells and non-target cells is utilized. In particular, loss of target cells during treatments can be reduced by labeling non-target cells at the filter used in the former purification step. Even though a plurality of types of target cells are present and different antigens are expressed in these cells, by labeling non-target cells, there are no limitations on the antigens of the target cells and non-target cells and target cells can be separated through labeling separation and the target cells can be selectively collected. It is conceivable that performing these steps as a series of flows in the above order makes it possible to separate and remove non-target cells and finally collect the target cells with a high purity while reducing loss of target cells. However, the present disclosure need not be interpreted as being limited to these mechanisms.

"Target cells" in the present disclosure refers to cells that are to be collected, detected, assayed, or analyzed. "Non-target cells" in the present disclosure refers to cells other than the cells that are to be collected, detected, assayed, or analyzed (i.e., cells that are not to be detected, assayed, or analyzed).

In one or more embodiments, the collection method of the present disclosure can be suitably utilized for a specimen containing a significantly smaller amount of target cells than non-target cells. A "specimen" is a sample that contains both target and non-target cells. In one or more embodiments, the method of the present disclosure can be particularly suitably utilized in a case where the specimen is a blood sample, the target cells are rare cells such as CTCs, and the non-target cells are white blood cells.

In one or more embodiments, the blood sample is a sample containing components constituting blood, and in one or more embodiments that are not particularly limited, examples thereof include blood, blood-derived materials containing blood cell components, body fluid and urine in which blood or blood-derived materials are mixed in, and a sample prepared from these. An example of blood is blood collected from an organism, and examples of the organism include a human and an animal (for example, a mammal) other than a human. Examples of the blood-derived materials containing blood cell components include samples that are separated or prepared from blood and contain blood cell components, and dilutions/concentrates thereof, such as blood cell fractions from which blood plasma is removed, blood cell concentrates, lyophilizates of blood or blood cells, samples obtained by subjecting whole blood to hemolysis treatment and removing red blood cell components, hemolyzed samples, centrifuged blood, spontaneously sedimented blood, washed blood cells, and a specific fraction. Among these, in one or more embodiments that are not limited, from the viewpoint of easy and quick treatment and suppressing damage to rare cells in blood, blood or a specimen derived from blood containing blood cell components is preferable as the sample containing the blood.

A "rare cell" is a cell which contains 1000 or fewer cells in 10 ml of a specimen obtained from a natural source or is present in the specimen obtained from a natural source in a ratio of 1% or less with respect to the number of non-target cells in the specimen. In one or more embodiments that are not particularly limited, "rare cells" are cells selected from the group consisting of cancer cells, circulatory tumor cells, vascular endothelial cells, vascular endothelial progenitor cells, cancer stem cells, epithelial cells, hematopoietic stem cells, mesenchymal stem cells, fetal cells, stem cells, and combinations thereof.

"Purification" in the present disclosure refers to increasing the ratio of target cells in a specimen containing target cells and non-target cells, or reducing the number or the ratio of non-target cells.

"Concentrate" in the present disclosure refers to increasing the ratio of target cells in a sample or gathering target cells in a specific region.

Also, with regard to "during any step among all of the steps" or "perform during a purification step and concentration step" in the present disclosure, "during a step" includes a period starting from preparation of this step to completion of this step.

According to the method of the present disclosure, in one or more embodiments, even in a case where the number of target cells in a specimen is 10 or less, target cells can be collected. Also, according to the collection method of the present disclosure, in one or more embodiments, it is possible to detect target cells from a specimen that contains a small amount of target cells, such as 10 ml of the specimen containing 100 target cells or less, and to detect target cells from a specimen that contains a small amount of target cells, such as 10 ml of the specimen containing 10 target cells or less. Also, a specimen whose ratio of target cells to all of the cells in the specimen (target cells/all cells) is 0.00000001% to 0.0003% before treatment (specimen) can be concentrated to a specimen whose ratio of target cells to all of the cells in a collection liquid obtained after the treatment is 0.1% or more, or higher than 0.1%, for example, 1% or more. According to the collection method of the present disclosure, in one or more embodiments, 8 mL of the specimen can be concentrated such that the amount of a suspension containing target cells obtained in the additional concentration step is 100 µL or less, or is less than 100 µL, for example, 50 µL or less.

"Small amount" in the present disclosure means that a small amount of a sample containing components of target cells is used when a small amount of a reaction liquid is used in genetic analysis. That is, in general, the genetic amplification is performed with a liquid amount of 50 µL to 100 µL, and the amount of the sample containing components of the target cells is smaller than the liquid amount. Also, in general, 1 ml to 10 ml of a blood specimen containing target rare cells and non-target cells is utilized, and thus "small amount" in the present disclosure means a significantly smaller amount compared to the liquid amount of the blood specimen treated in the filtering step.

### Collection method of present disclosure

In one aspect, the present disclosure relates to a method for collecting rare cells in a specimen containing target rare cells and non-target cells (a collection method of the present disclosure).

The collection method of the present disclosure includes a filtering step, a labeling step, a separation/collection step, and a capture step below.

Filtering step: filtering a specimen containing target rare cells and non-target cells with a filter capable of capturing the target rare cells

Labeling step: labeling the non-target cells among the rare cells and the non-target cells that were captured by the filter, at the filter

Separation/collection step: removing the labeled non-target cells from a solution containing the rare cells and the labeled non-target cells, which collected the filter, to collect the solution containing the rare cells.

Capture step: capturing the rare cells from the solution containing the rare cells that was collected in the collection step

According to the collection method of the present disclosure, the above-described filtering step, labeling step, separation/collection step, and capture step are performed in this order, and thus, in one or more embodiments, the effects below can be achieved.

Performing filtering treatment with the filter before labeling treatment makes it possible to remove impurities from the specimen and reduce the amount of non-target cells, and thus to increase the reactivity between non-target cells and labeling substances and reduce the cost required for labeling. Also, because liquid surrounding cells can be replaced while cells are captured by filtering treatment, the labeling treatment can be performed in an environment that is suitable for reaction between non-target cells and labeling substances, while loss of target cells is reduced.

The labeling treatment is performed at the filter that has captured cells, that is, a step of collecting target cells from the filter is not included between the filtering step and the labeling step, and thus it is possible to reduce loss of target cells that will occur during collection from the filter.

Because the labeled non-target cells are separated and the target cells are selectively collected before target cells are captured (or concentrated), it is possible to efficiently separate the labeled non-target cells and increase the purity of a collection liquid (for example, a concentrated liquid) that is obtained finally

### Purification step and concentration step

The purification step and concentration step can be performed using a method in which a difference in physical properties between target rare cells and non-target cells is utilized. Such a method includes a step of filtering the specimen with a filter that has a plurality of through holes and is capable of capturing the target rare cells or a step of removing a proportion of the non-target cells in the specimen by filtering the specimen with a filter that has a plurality of through holes and is capable of selectively capturing the target rare cells, and a step of separating the specimen into at least two layers, namely a layer containing the target rare cells and a layer other than the layer containing the target rare cells with a density gradient method so as to obtain a cell suspension containing the target rare cells, for example. An example of the former is a step of removing a portion of the non-target cells in the specimen through filtering the specimen containing the target cells and the non-target cells with a filter that has a plurality of through holes and is capable of capturing target rare cells, and preferably is a step of removing a portion of non-target cells in a specimen through filtering with a filter capable of selectively capturing target cells. In this case, in the purification step and concentration step, the target cells in the specimen are captured by the filter. Also, in one or more embodiments, a proportion of the non-target cells in a specimen may be captured by the filter. As an example of the latter, a step in which a density gradient method that is known by persons skilled in the art can be used appropriately.

The purification step and concentration step will be described using a case in which the above-described filter is used (a filtering step in the collection method of the present disclosure), as an example. In one or more embodiments, an example of the filter that has a plurality of through holes and is capable of capturing target cells in a specimen is a slit-shaped filter having a plurality of through holes. In one or more embodiments, examples of the shape of the through holes include a rounded rectangle and an ellipse. In one or more embodiments, examples of the material of the filter include plastic materials such as parylene and polycarbonate and metals such as nickel, SUS, gold, silver, copper, aluminum, tungsten, and chromium.

In one or more embodiments, an example of the filter is a filter capable of capturing rare cells in a specimen, and for example, a filter capable of separating target cells and non-target cells through filtering, utilizing a difference in the deformability or the viscosity of target cells and non-target cells can be utilized suitably. Also, in one or more embodiments, the filter preferably lets the majority of white blood cells pass through and is capable of capturing rare cells, and is more preferably capable of capturing CTCs in a specimen, and an example thereof is a filter capable of capturing at least SNU-1, SW620, or both of these, for example, and is not limited to these. In one or more embodiments, a conventionally known filter or a filter that will be developed later and can capture rare cells can be used as the filter. In one or more embodiments, examples of the filter include the filter disclosed in WO 2011/108454 and the filter disclosed in JP 2016-057313A. Also, examples of the filter include a sheet-shaped single membrane including multiple through holes that are each an ellipse having a minor axial diameter of 1 µm to 50 µm, a perfect circle, a rectangle, a rectangle with round corners, slit shape, or the like, and are preferably membranes other than nonwoven fabric.

In one or more embodiments, from the viewpoint of improving the capturing rate for the rare cell having a high deformability and/or the viewpoint of suppressing bending, distortion, the filter may include holes having an average minor axis diameter of about 5 µm or more and less than about 10 µm and an average major axis diameter of 10 µm or more and 5000 µm or less. In one or more embodiments, from the viewpoint of improving the capturing rate for the rare cell having a high deformability, the average major axis diameter may be about 40 µm or more, 50 µm or more, 60 µm or more, 70 µm or more, or 80 µm or more. From the viewpoint of suppressing bending, distortion, and the like of the filter to improve the capturing rate for the rare cell, the average major axis diameter may be about 5000 µm or less, 4000 µm or less, 3000 µm or less, 2000 µm or less, 1000 µm or less, 970 µm or less, 900 µm or less, 800 µm or less, 700 µm or less, 600 µm or less, 500 µm or less, 400 µm or less, 300 µm or less, 200 µm or less, 150 µm or less, 120 µm or less, 100pm or less, 90 µm or less, or 80 µm or less.

In one or more embodiments, there is no particular limitation on the filtering area of the filter, and the filtering area is 5 mm² or more or 10 mm² or more, and 10,000 mm² or less, 2,000 mm² or less, 1,000 mm² or less, 500 mm² or less, 200 mm² or less, 150 mm² or less, 100 mm² or less, 80 mm² or less, 50 mm² or less, 40 mm² or less, 30 mm² or less, or 25 mm² or less. "Filtering area of filter" in the present disclosure refers to the area of a portion of the entire area of the filter that comes into contact with the specimen or the suspension (i.e., the area of a portion to which liquid is fed, among the entire area of the filter).

Although there is no particular limitation on the number of through holes of the filter, in one or more embodiments, the number of through holes can be set as appropriate in accordance with a blood amount per hole in a region through which a sample passes. In one or more embodiments, the filter has through holes at a hole density of 40 holes/mm² or more and 2000 holes/mm² or less. From the viewpoint of further increasing the ratio of capturing rare cells having a high deformability, suppressing the filtering area, and/or efficiently collecting rare cells from the filter, the hole density (the number of holes in 1 mm²) is 45 holes/mm² or more, 60 holes/mm² or more, 70 holes/mm² or more, 90 holes/mm² or more, 200 holes/mm² or more, 300 holes/mm² or more, 400 holes/mm² or more, 500 holes/mm² or more, 600 holes/mm² or more, or 700 holes/mm² or more, and from similar viewpoints, 40000 holes/mm² or less, 10000 holes/mm² or less, 4000 holes/mm² or less, 1500 holes/mm² or less, 1000 holes/mm² or less, 900 holes/mm² or less, or 800 holes/mm² or less.

In one or more embodiments, the thickness of the filter is 1 to 1000 µm, 1 to 100 µm, 1 to 50 µm, 1 to 20 µm, or preferably 1 to 10 µm, and more preferably 1 to 8 µm.

From the viewpoint of further increasing the rare cell capture ratio, suppressing the filtering area, and/or efficiently collecting rare cells from the filter, in one or more embodiments, the opening ratio of the filter is 10% or more and 60% or less. From similar viewpoints, the opening ratio of the filter is 15% or more, 20% or more, 25% or more, 30% or more, 31% or more, 35% or more, or 40% or more, and from similar viewpoints, 55% or less or 45% or less.

From the viewpoint of increasing the cell capture ratio, in one or more embodiments, the flow velocity of the specimen is 0.01 mm/sec or more, 0.05 mm/sec or more, or 0.1 mm/sec or more per opening area, and from a similar point, the flow velocity is 100 mm/sec or less, 50 mm/sec or less, 25 mm/sec or less, 10 mm/sec or less, 5 mm/sec or less, or 2 mm/sec or less per opening area.

The amount of the passing specimen (e.g., the amount of a blood specimen that passes through the filter) is 0.07 ml or more, 0.25 ml or more, 0.5 ml or more, 1 ml or more, an amount larger than 1 ml, 2 ml or more, 3 ml or more, or 4 ml or more, with respect to a filtering area of 5 mm² to 10,000 mm², for example. Also, in one or more embodiments, from the viewpoint of maintaining the rare cell capture ratio, the amount of the passing specimen is 6 L or less, 4 L or less, 2 L or less, 400 mL or less, 200 mL or less, 100 mL or less, 80 mL or less, 50 mL or less, 30 mL or less, 25 mL or less, 12 mL or less, 11 mL or less, 10 mL or less, 9 mL or less, or 8 mL or less, with respect to a filtering area of 5 mm² to 10,000 mm². Alternatively, in one or more embodiments, from the viewpoint of treating a large amount of the sample at once, capturing cells that tend to deform, and maintaining the rare cell capture ratio, the capacity of the filter is more than 0.2 ml and less than 130 ml or 130 ml or less with respect to a filtering area of 15 mm² to 200 mm², and from similar viewpoints, the capacity is 0.8 ml to 90 ml, 0.8 ml to 60 ml, 2 ml to 50 ml, or 2 ml to 40 ml.

From the viewpoint of increasing the cell capture ratio, in one or more embodiments, the pressure of the passing specimen is 0.1 kPa or more or 0.2 kPa or more, and 2.6 kPa or less, 1.5 kPa or less, 1.3 kPa or less, 1.0 kPa, or 0.5 kPa or less. "Pressure of a passing specimen" in the present disclosure refers to a "difference in pressure of the whole system from an inlet of the system to its outlet", and for example, refers to a "difference in pressure of a system that extends from a tank in which the specimen is disposed to a waste liquid tank and includes a flow channel extending from the tank to the waste liquid tank". Thus, a pressure that is greater than or equal to the above-described pressure may be used depending on the structure of the flow channel. The pressure of a passing liquid (e.g., a difference in pressure of the system) can be measured using a commercially available pressure gauge with a known method, or can be calculated based on a relationship between the flow rate and the structure of the flow channel.

Alternatively, rare cells can be captured by feeding a blood specimen at an average flow velocity of 1 mm/min or more and 600 mm/min or less per hole (one hole) of the filter. In one or more embodiments, the conditions under which the blood specimen is fed, the flow velocity is 2 mm/min or more and 300 mm/min or less, 3 mm/min or more and 100 mm/min or less, 4 mm/min or more and 80 mm/min or less, or 4 mm/min or more and 40 mm/min or less. Liquid feeding conditions may be set through conversion based on an average flow rate. As a calculation method, the average flow rate per hole can be obtained by measuring the flow rate (combined average flow rate of all of the holes) of a filtering waste liquid outlet per unit of time, or filtering time per unit of amount, and dividing the resulting value by the number of holes. Also, the average flow velocity can be obtained by dividing the average flow rate by the area of holes. Liquid may be fed at a flow velocity or a flow rate that is in a range of the above-described average flow velocity or average flow rate per hole. In one or more embodiments, the average flow rate per hole may be calculated by dividing the flow rate (combined average flow rate of all of the holes) of the filtering waste liquid outlet by the area of all of the holes.

The filtering treatment may be performed such that the total number of non-target cells included in the cell suspension collected after blood has undergone the filtering step or the filtering step and the labeling step is preferably 100000 or less, 50000 or less, 10000 or less, or 6000 or less.

### Labeling step (labeling treatment)

In the labeling step, for example, the non-target cells that were captured by the filter together with the target cells in the former purification step (for example, the filtering step) can be labeled at the filter. In one or more embodiments, an example of the labeling method is magnetic labeling.

In one or more embodiments, magnetic labeling can be performed by binding binding molecules of the non-target cells and substances fixed to the surfaces of the magnetic particles (e.g., substances that specifically react with binding molecules of non-target cells) so as to fix the magnetic particles to the non-target cells. In one or more embodiments, the substances that are fixed to the magnetic particles can be determined as appropriate in accordance with the binding molecule of the non-target cells. In one or more embodiments, examples of the magnetic particle include a magnetic particle having a surface to which avidin is fixed, a magnetic particle having a surface to which streptavidin is fixed, a magnetic particle having a surface to which neutravidin is fixed, a magnetic particle having a surface to which biotin is fixed, a magnetic particle having a surface to which a biotin derivative is fixed, a magnetic particle having a surface to which an antibody is fixed, and a magnetic particle having a surface to which an antigen is fixed. There is no particular limitation on the size of the magnetic particle.

From the viewpoint of increasing the labeling efficiency with magnetic particles, in one or more embodiments, in the labeling step, a suspension containing magnetic particles is supplied to the surface of the filter on which target cells are captured, and a portion of the supplied suspension is fed from the surface of the filter to the other surface of the filter using a liquid feeding means such as a pump, for example. With the labeling step of the present disclosure, a portion of the suspension is actively fed toward the surface of the filter (i.e., the lower surface of the filter) that is opposite to the cell capture surface, and this is different from a phenomenon in which liquid unintentionally permeates or diffuses through the filter and reaches the vicinity of the lower surface of the filter. In the labeling step of the present disclosure, for example, by supplying the suspension to the surface of the filter on which target cells are captured, and feeding a portion of the supplied suspension to the other surface of the filter, a state is created in which liquid containing magnetic particles is in contact with both surfaces of the filter, and the liquid containing magnetic particles is preferably reversely fed from the other surface of the filter toward the surface of the filter on which the target cells are captured. In one or more embodiments, feeding of the suspension may include feeding a portion of the suspension toward the lower surface side of the filter such that the upper liquid surface and the lower liquid surface of the liquid containing magnetic particles sandwich the filter. In one or more embodiments, this liquid feeding can be performed such that liquid containing magnetic particles is held in the vicinity of the opposite surface of the filter (i.e., the lower surface of the filter). In one or more embodiments, this liquid feeding is performed by drawing the suspension with a pump from the opposite surface side (i.e., the lower surface of the filter) such that the suspension passes through the filter and the liquid containing magnetic particles comes into contact with both surfaces of the filter.

In one or more embodiments, the labeling step may include performing treatment in which binding molecules are fixed to non-target cells, before the suspension containing magnetic particles is fed.

### Additional purification

In one or more embodiments, additional purification includes a step of separating the suspension containing target cells and non-target cells that were obtained in the former purification step and concentration step so as to obtain a suspension containing target cells.

The additional purification step can be performed using a method in which a difference in biological properties between target rare cells and non-target cells is utilized, and/or a method in which a difference in physical properties between target rare cells and non-target cells is utilized. Examples of such a method include at least one of a method in which magnetically-labeled cells are separated from non-magnetically-labeled cells, a method in which target rare cells are separated from cells other than the target rare cells through suction, a method in which target rare cells are separated from cells other than the target rare cells through flow cytometry, and a method in which dielectrophoresis is used. In the method in which a difference between biological properties is utilized and the method in which a difference in physical properties between target rare cells and non-target cells is utilized, in one or more embodiments, an example of the additional purification step is a step in which technology for deflecting the target rare cells and the non-target cells in different directions in a flow channel is used. For example, in the method by which labeled cells and non-labeled cells are separated, such an additional purification step includes supplying a suspension containing labeled cells and non-labeled cells from one end of a flow channel, deflecting the labeled cells in the flow channel in a direction that is different from the direction in which the non-labeled cells are deflected, fixing the labeled cells onto an inner wall surface of the flow channel, ejecting the suspension in the flow channel from another end of the flow channel by introducing a gas phase into the flow channel from one end of the flow channel in a state in which the labeled cells are fixed to the inner wall surface of the flow channel, and collecting the non-labeled cells. For example, the additional purification step can be performed by supplying the suspension collected after the labeling step to the flow channel and fixing the labeled non-target cells onto the inner wall surface of the flow channel. By "fixing7' is meant immobilizing the non-target cells, e.g. on a solid support such as a surface of a flow channel.

In one or more embodiments, the additional purification step can be performed through magnetic separation. In some embodiments, the separation/collection step in the method of the present disclosure includes the magnetic separation. In one or more embodiments, additional purification (for example, the separation/collection step in the collection method of the present disclosure) includes removing the labeled non-target cells from a solution containing the target cells and the non-target cells that were magnetically labeled in the labeling step through magnetic separation, and selectively collecting the target cells.

From the viewpoint of highly precisely separating the target cells that are not magnetically labeled and the non-target cells that are magnetically labeled, in one or more embodiments, the additional purification step (e.g., the separation/collection step in the collection method of the present disclosure) preferably includes supplying a suspension collected after the labeling step into the flow channel in which a magnetic field is formed, and fixing the magnetically labeled non-target cells onto the inner wall surface of the flow channel. From the viewpoint of further increasing the separation precision, the additional purification step preferably includes deflecting, in the flow channel, the magnetically labeled non-target cells in a direction that is different from the direction in which the target cells are deflected, and fixing the magnetically labeled non-target cells onto the inner wall surface of the flow channel in the deflection direction. Also, from the viewpoint of reducing loss of target cells, the additional purification step is preferably performed by ejecting the suspension in the flow channel from the other end of the flow channel by introducing a gas phase into the flow channel from one end of the flow channel.

"Gas phase" in the present disclosure refers to a phase constituted by gas. In one or more embodiments, the gas phase can be introduced so as to eject the suspension in the flow channel together with non-labeled cells with pressure of the introduced gas phase from the other end of the flow channel. In one or more embodiments, the gas phase can be introduced so as to form a gas-liquid interface between the suspension in the flow channel and the introduced gas phase, and so as to move this gas-liquid interface from one end (e.g., inlet) of the flow channel toward the other end (e.g., outlet). In one or more embodiments, the gas-liquid interface occupies a certain region, which is not a point or line, between the inner wall surface of the flow channel and the liquid. Also, points at which the gas-liquid interface and the inner wall surface of the flow channel are in contact with each other form a continuous line in one or more embodiments. In one or more embodiments, air, oxygen, nitrogen, argon, carbon dioxide, or the like can be used as gas.

In one or more embodiments, the gas phase can be introduced by a pressure generation mechanism or the like that is arranged at one end of the flow channel. In one or more embodiments, examples of the pressure generation mechanism include a syringe pump, a tube pump, a booster pump, and a vacuum pump.

From the viewpoint of the fact that a stable gas-liquid interface can be formed and precision in separation of non-labeled cells can be increased, and the fact that labeled cells that are fixed to the inner wall surface of the flow channel are kept fixed thereon, in one or more embodiments, the flow rate of the gas phase is 25 µl/min or more, 50 µl/min or more, or 75 µl/min or more, and 500 µl/min or less or 250 µl/min or less.

### Additional concentration step

The additional concentration step can also be performed after the additional purification step, for example. In the additional concentration step, the suspension obtained in the additional purification step is concentrated, for example. Such an additional concentration step can be performed using the method in which a difference in biological properties between the target rare cells and the non-target cells is utilized, and/or a method in which a difference in physical properties between the target rare cells and the non-target cells is utilized. Examples of such a method include at least one of a method by which magnetically-labeled cells are separated from non-magnetically-labeled cells, a method by which target rare cells are separated from cells other than the target rare cells through suction, a method by which target rare cells are separated from cells other than the target rare cells through flow cytometry, and a method in which dielectrophoresis is used. In the concentration step, an example of the method in which a difference in biological properties is utilized, and/or the method in which a difference in physical properties between the target rare cells and the non-target cells is utilized is a method in which dielectrophoresis is utilized. In some embodiments, the capture step in the method of the present disclosure includes capturing by using the method in which a difference in physical properties between the target rare cells and the non-target cells is utilized. "Capture" as referred to herein means to concentrate, accumulate or aggregate together the cells, e.g. in a small space. This does not necessarily require attachment or association with a collection vessel or material. As described hereinbefore the capture may be performed in various ways such as by flow cytometry, suction or dielectrophoresis.

That is, in one or more embodiments, it is preferable that the suspension is concentrated (for example, the capture step in the collection method of the present disclosure) through dielectrophoresis because the target cells can be collected in a small space. In one or more embodiments, the suspension can be concentrated through dielectrophoresis by introducing the suspension obtained in the additional purification step into a chamber in which dielectrophoresis occurs, and capturing the target cells in the chamber through dielectrophoresis.

From the viewpoint of suppressing a decrease in the occurrence of polarization in target cells, reducing damage of flowing electric current to the target cells, or further increasing the capture ratio through dielectrophoresis, the solvent of the suspension obtained in the additional purification step (e.g., the separation/collection step) is desired to have an electrical conductivity that is as low as possible. From similar viewpoints, in one or more embodiments, the solvent preferably contains an electrolyte in a small amount. From similar viewpoints, in one or more embodiments, the solvent is preferably a non-electrolytic isotonic solution such as a sucrose isotonic solution. Also, from the viewpoint of reducing inhibition of gene amplification, the solvent is preferably a non-electrolytic solution such as a sucrose isotonic solution.

In the present disclosure, in one or more embodiments, a step A of removing a proportion of non-target cells in a specimen by filtering the specimen with a filter that has a plurality of through holes and is capable of selectively capturing the target rare cells is performed as the purification step and the concentration step, and next, a step B performed using a method by which the magnetically-labeled cells are separated from non-magnetically-labeled cells is performed as the additional purification step, next, a step performed using a method in which dielectrophoresis is used is performed as the additional concentration step, and in the step A, labeling treatment is performed on the cells for the step B in the step A. With this mode, the target cells can be collected from the specimen containing the target rare cells and the non-target cells with only these four steps with a high purity and a small amount.

### Assay or detection method of present disclosure

In one aspect, the present disclosure relates to a method for assaying or detecting target cells from a specimen containing target cells and non-target cells (the assay or detection method of the present disclosure).

In one or more embodiments, the assay or detection method of the present disclosure includes a step of assaying or detecting cells during or after implementation of the collection method of the present disclosure.

In one or more embodiments, the assay or detection method of the present disclosure can be performed similarly to the collection method of the present disclosure.

The assay or detection method of the present disclosure includes the assay step or the detection step of assaying or detecting cells in the suspension obtained in the concentration step (e.g., the capture step). The assay or detection step includes imaging target cells in the suspension and analyzing the image obtained through imaging. Also, the assay or detection step includes collecting the suspension obtained in the concentration step and analyzing or detecting the genes of the cells in the suspension. In one or more embodiments, examples of the gene include EGFR, ALK, RAS, BRAF, PIK3CA, SYT-SSX, IDH1, JAK2, CALR, MPL, BCR-ABL, c-kit, NPM1, MYD88, RHOA, ABCG2, HER2, RET, ROS1, EML4-ALK, MEK1, MET, KIF5B-RET, and combinations thereof.

### Collection system of present disclosure

In one aspect, the present disclosure relates to a system for collecting rare cells (the collection system of the present disclosure).

In one or more embodiments, the collection system of the present disclosure includes a purification/concentration unit (e.g., a filtering unit) that implements the purification step and concentration step (e.g., the filtering step) and an additional purification/concentration unit (e.g., a separation/collection unit and a capture unit) that implements a purification step and concentration step (e.g., the separation/collection step and the capture step) in addition to the purification step and concentration step, and the system is configured to be capable of implementing, during any step that is implemented in the system, the labeling treatment on the cells for the purification step or the concentration step that will be performed after said step.

"In addition to" above means that a pair of steps, such as the purification step and concentration step, is implemented at least two times, and does not mean forming a subordinate relationship between the former purification step and concentration step and the latter purification step and concentration step.

In one or more embodiments, in the collection system of the present disclosure, the purification/concentration unit has a physical separation means for utilizing a difference in physical properties between target rare cells and non-target cells, and the additional purification/concentration unit has a biological separation means of detecting a difference in biological properties between target rare cells and non-target cells and/or a physical separation means for detecting a difference in physical properties between target rare cells and non-target cells.

In the collection system of the present disclosure, in the purification/concentration unit, a portion that performs purification and a portion that performs concentration may be the same, integrated with, or separate from each other. Also, in the additional purification/concentration unit, a portion that performs purification and a portion that performs concentration may be the same, integrated with, or separate from each other.

The purification/concentration unit can be provided with a separation unit, for example, and depending on the cases, may have a liquid feeding means capable of feeding a specimen to the separation unit. Also, the purification/concentration unit may include a liquid feeding means capable of feeding a suspension containing a label such as magnetic particles used in the labeling step and a washing liquid. In one or more embodiments, the purification/concentration unit can perform the purification/concentration step in the collection method of the present disclosure, or the labeling step as desired.

In one or more embodiments, the additional purification/concentration unit has a flow channel through which the suspension can be supplied, a magnetic field emission body disposed in/on at least one of the upper portion and the side surface of the flow channel, and an introduction means for ejecting the suspension in the flow channel to the outside of the flow channel, for example. By generating a magnetic field from the magnetic field emission body, magnetically-labeled white blood cells can be fixed to the inner wall surface of the flow channel corresponding to a location at which the magnetic field emission body is disposed. In one or more embodiments, the magnetic field emission body may be arranged extending over the longitudinal direction of the flow channel, or may be arranged in at least a portion in the longitudinal direction of the flow channel. In one or more embodiments, the number of magnetic field emission bodies may be one, or two or more. In one or more embodiments, examples of the magnetic field emission body include a magnet and an electromagnet. In one or more embodiments, the introduction means may be capable of introducing a liquid phase or a gas phase. In one or more embodiments, examples of the introduction means for introducing the gas phase include pressure generation mechanisms such as a syringe pump, a tube pump, a booster pump, and a vacuum pump.

In one or more embodiments, the capture unit (e.g., the additional purification/concentration unit) has a flow channel chamber into which a suspension can be supplied and an electric field generation means for causing dielectrophoresis, and the electric field generation means is disposed in the flow channel chamber, for example. From the viewpoint of improving ease of observation (visibility), the electric field generation means is preferably disposed on the bottom surface of the flow channel chamber. In one or more embodiments, an example of the electric field generation means is a counter electrode for dielectrophoresis.

In one or more embodiments, the chamber of the capture unit may be connected to the flow channel of the collection unit such that the suspension ejected from the flow channel of the collection unit can be directly supplied to this chamber.

Hereinafter, one embodiment that is not limited of the collection/detection method of the present disclosure will be described.

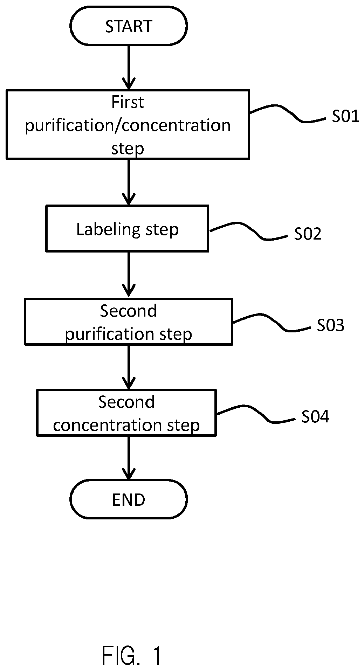

FIG. 1 is a flowchart showing one embodiment of the collection/detection method of the present disclosure.

First, in the filtering step (e.g., first purification/concentration step) (step S01), rare cells (target cells) and the other cells (non-target cells) in blood are roughly separated using a filter having a plurality of through holes. Rough separation can be performed by feeding blood to the filter having a plurality of through holes. The filter is provided with a plurality of through holes in which rare cells can be captured. The feeding conditions are as follows. Although this filter can capture rare cells, blood contains a large amount of white blood cells (e.g., 30 million to 70 million cells in 10 ml), and the diameter of white blood cells is approximately equal to or larger than that of rare cells, and thus a portion of white blood cells in a specimen will be captured by the filter together with rare cells through filtering treatment. In this example, in the specimen containing target rare cells and non-target cells such as white blood cells, the ratio of the target rare cells can increase and the target rare cells in the specimen can be accumulated on the filter through the filtering treatment, and thus in this example, the filtering step (e.g., the purification/concentration step) is performed by only one unit in the present disclosure.

From the viewpoint of increasing the reactivity of a reagent to white blood cells, red blood cells remaining on the filter may be removed through treatment by supplying a hemolytic agent before the labeling step (step S02).

In the labeling step (step S02), white blood cells captured by the filter in the filtering step (step S01) are magnetically labeled with magnetic particles. From the viewpoint of reducing loss of rare cells and increasing the collection ratio, magnetic labeling is performed at the filter.

From the viewpoint of increasing the reactivity between magnetic particles and white blood cells, white blood cells may be subjected to immunostaining with an antibody before supply of the suspension. Examples of the antibody include antibodies bound to binding molecules. In one or more embodiments, the binding molecule is biotin or the like. Also, for example, magnetic particles to which a substance that specifically reacts with the above-described binding molecules is fixed may be used as the magnetic particles. In one or more embodiments, examples of the specifically reacting substance include proteins such as streptavidin and neutravidin.

Then, magnetic labeling is performed by feeding the suspension containing magnetic particles to the filter on which rare cells and white blood cells are captured. Magnetic labeling is performed by feeding this suspension such that both main surfaces of the filter are sandwiched between an upper liquid surface and a lower liquid surface of the suspension containing magnetic particles, stirring the liquid on the filter after feeding in the reverse direction, and then letting magnetic particles and white blood cells react with each other. From the viewpoint of increasing the reactivity between magnetic particles and white blood cells, these operations may be repeated.

From the viewpoint of increasing the efficiency of detection of rare cells after separation collection, rare cells may be subjected to immunostaining with an antibody after magnetic labeling. Examples of the antibody include antibodies that recognize antigens expressed in CTCs, such as Cytokeratin. From the viewpoint of causing antibodies to react with antigens in rare cells, fixation treatment and membrane permeation treatment may be performed before labeling with the antibodies.

In a separation/collection step (e.g., second purification step) (step S03), the suspension obtained in the labeling step S02 is magnetically separated. Magnetic separation is performed by fixing the magnetically-labeled white blood cells to the inner wall surface of the flow channel by supplying the collected suspension in the flow channel in which the magnetic field is formed, and ejecting rare cells to the outside of the flow channel in this state. From the viewpoint of increasing the efficiency of ejection of rare cells, the magnetic field is preferably formed such that white blood cells are fixed to wall surfaces other than the bottom surface of the flow channel. The rare cells can be ejected by introducing a liquid phase or a gas phase into the flow channel. From the viewpoint of further increasing the efficiency of separation of rare cells and white blood cells, the rare cells are preferably ejected by introducing a gas phase. The rare cells may be ejected by directly supplying the rare cells ejected from the flow channel to the chamber used in a capture step (second concentration step) (step S04) that will be described later.

In the capture step (e.g., second concentration step) (step S04), the suspension containing the rare cells that has undergone the separation/collection step (the second purification step) (step S03) is concentrated. Because target cells can be aggregated in a small space, the suspension is concentrated through dielectrophoresis. The suspension is concentrated through dielectrophoresis by introducing the suspension obtained in the separation/collection step (step S03) into the chamber in which dielectrophoresis occurs, and ejecting liquid (e.g., the solvent of the suspension) to the outside of the chamber while capturing the target cells in the chamber through dielectrophoresis.

The present disclosure may relate to one or more embodiments below.
[1] A collection method for collecting target rare cells from a specimen containing the target rare cells and non-target cells, with a high purity and a small amount, the method including:
   a purification step and concentration step, and an additional purification step and additional concentration step;
   in which during any step among all of the steps, labeling treatment is performed on cells for a purification step or concentration step that will be performed after the labeling treatment.
[2] The collection method according to [1],
   in which the purification step and concentration step are performed using a method in which a difference in physical properties between the target rare cells and the non-target cells is utilized, and
   the additional purification step and additional concentration step are performed using a method in which a difference in biological properties between the target rare cells and the non-target cells is utilized and/or a method in which a difference in physical properties between the target rare cells and the non-target cells is utilized.
[3] The collection method according to [1] or [2],
   in which the purification step and concentration step include at least one of
   a step of removing a proportion of the non-target cells in the specimen by filtering the specimen with a filter that has a plurality of through holes and is capable of selectively capturing the target rare cells, and
   a step of separating the specimen into at least two layers, namely, a layer containing the target rare cells and a layer other than the layer containing the target rare cells using a density gradient method, and obtaining a cell suspension containing the target rare cells.
[4] The collection method according to any of [1] to [3],
   in which the additional purification step and additional concentration step are performed using at least one of a method by which magnetically-labeled cells are separated from non-magnetically-labeled cells, a method by which target rare cells are separated from cells other than the target rare cells through suction, a method by which the target rare cells are separated from the cells other than the target rare cells through flow cytometry, and a method in which dielectrophoresis is used.
[5] The collection method according to any of [1] to [4],
   in which the labeling treatment is for labeling, at the filter, the non-target cells among the cells captured by the filter.
[6] The collection method according to any of [1] to [5],
   in which the labeling treatment is performed during the purification step and concentration step.
[7] The collection method according to any of [1] to [6],
   in which the cells that are labeled through the labeling treatment are white blood cells.
[8] The collection method according to any of [1] to [7],
   in which 1 or more and 100 or fewer of the target rare cells in the specimen are present in 10 ml of the specimen.
[9] The collection method according to any of [1] to [8],
   in which 1 or more and 10 or fewer of the target rare cells in the specimen are present in 10 ml of the specimen.
[10] The collection method according to any of [1] to [9],
   in which a ratio of the target rare cells in the obtained collection liquid to all of the cells is 0.1 % or more and 100% or less.
[11] The collection method according to any of [1] to [10],
   in which a ratio of the target rare cells in the obtained collection liquid to all of the cells is 1% or more and 100% or less.
[12] The collection method according to any of [1] to [11],
   in which an amount of the obtained collection liquid is 1 pL or more and 100 µL or less.
[13] The collection method according to any of [1] to [12],
   in which an amount of the obtained collection liquid is 1 pL or more and 50 µL or less.
[14] An assay or detection method including:
   a step of assaying or detecting cells during implementation or after completion of the collection method according to any one of [1] to [13].
[15] The assay or detection method according to [14],
   in which the assay or detection step includes imaging the target rare cells and analyzing an image obtained through imaging.
[16] The assay or detection method according to [14] or [15],
   in which the assay or detection step includes analyzing or detecting genes of the target rare cells.
[17] A rare cell collection system for collecting target rare cells from a specimen containing the target rare cells and non-target cells, with a high purity and a small amount, the system including:
   a purification/concentration unit that implements a purification step and concentration step; and
   an additional purification/concentration unit that implements a purification step and concentration step, in addition to the purification step and concentration step,
   in which the system is configured to be capable of implementing, during any step that is implemented in the system, labeling treatment on cells for a purification step or a concentration step that will be performed after the step.
[18] The rare cell collection system according to [17],
   in which the purification/concentration unit has a physical separation means for utilizing a difference in physical properties between the target rare cells and the non-target cells, and
   the additional purification/concentration unit has a biological separation means for utilizing a difference in biological properties between the target rare cells and the non-target cells and/or a physical separation means for utilizing a difference in physical properties between the target rare cells and the non-target cells.
[19] The rare cell collection system according to [17] or [18],
   in which the purification/concentration unit includes at least one of a filter that has a plurality of through holes and is capable of selectively capturing the target rare cells, and a density gradient apparatus.
[20] The rare cell collection system according to any of [17] to [19],
   in which the additional purification/concentration unit includes at least one of a magnetic field generation apparatus, a dielectrophoretic apparatus, a suction apparatus, and a flow cytometry.
[21] The rare cell collection system according to any of [17] to [20],
   in which the purification/concentration unit is configured to be capable of labeling, at the filter, the non-target cells among the cells captured by the filter.

### Examples

Hereinafter, the present disclosure will be further described using an Example. However, the present disclosure is not interpreted as being limited to the Example below.

### Cell capture/staining apparatus

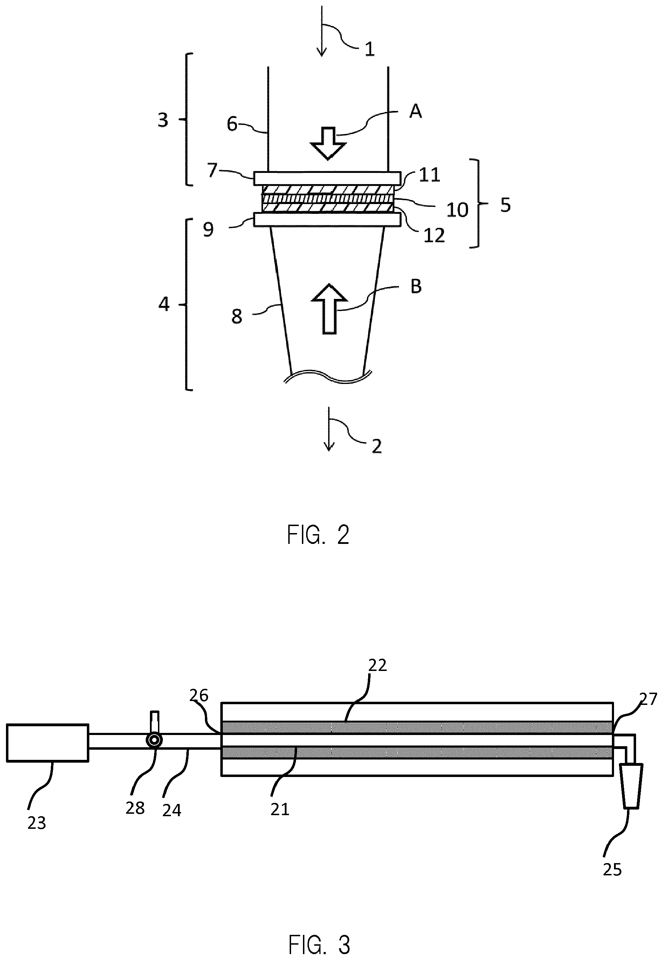

A cell capture/staining apparatus shown in FIG. 2 was prepared. The cell capture/staining apparatus in FIG. 2 is provided with a supply port 1 for supplying a suspension containing magnetic beads, a washing liquid, a staining liquid, or the like, an ejection port 2 for ejecting waste liquid such as a washing liquid, or the like, a device upper portion 3, a filter portion 5 including a filter 10, a device lower portion 4, and a liquid feeding mechanism (not shown) capable of feeding liquid in an arrow A direction and an arrow B direction. The device upper portion 3 has a flow channel 6 and a support member 7 for fixing the filter portion 5, and the flow channel 6 is continuous with the supply port 1. The device lower portion 4 has a flow channel 8 and a support member 9 for fixing the filter portion 5, and the flow channel 8 is continuous with the ejection port 2. The filter portion 10 is disposed between the device upper portion 3 and the device lower portion 4 and is fixed by double-sided tapes 11 and 12. The filter 10 is provided with a plurality of through holes, and a filter having properties below was used as the filter 10.

### Properties of filter

- Hole minor axial diameter: 6.5 µm, major axial diameter: 88 µm, area: 572 µm², shape: slit
- Pitch between centers of holes: 14 µm × 100 µm (minor axial diameter side × major axial diameter side)
- Hole density: 714 holes/mm²
- Opening ratio: 41%
- Membrane thickness: 5 µm
- Material: nickel
- Filtering area: 28 mm²

### Magnetic separation apparatus

A magnetic separation apparatus shown in FIG. 3 was prepared.

A syringe pump 23 was connected to one end 26 of a Safeed™ tube 21 (having an inner diameter of 3.1 mm, a tube length of 26 cm, and a volume of 2 cm³) via a tube 24 and a syringe (10 mL), and a container 25 for collecting liquid ejected from the Safeed™ tube was disposed at another end 27. A three-way valve 28 was disposed in the tube 24. A magnet (neodymium magnet (N40, square shape, 200×15×5 (mm), 5 mm magnetization direction, a surface magnetic flux density of 229 mT)) was disposed on a side surface of the Safeed™ tube.

### Dielectrophoretic apparatus

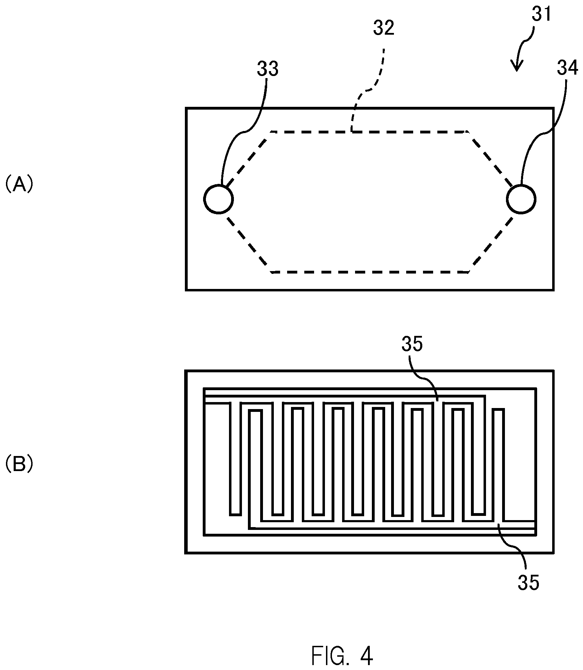

A dielectrophoretic apparatus shown in FIG. 4 was prepared. In FIG. 4, (A) is a schematic diagram of a dielectrophoretic apparatus 31 from the top, and (B) shows a situation in which comb electrodes 35 are disposed on a bottom surface of a chamber 32.

In the dielectrophoretic apparatus in FIG. 4, the microdevice 31 has an inlet 33, the chamber 32, an outlet 34, and the comb electrodes 35. The inlet 33 and the outlet 34 are formed on an upper surface of the dielectrophoretic apparatus 31 and connected to the chamber 32 formed in the longitudinal direction along the bottom surface of the dielectrophoretic apparatus 31. The comb electrodes 35 are formed on the upper surface of a substrate that constitutes the bottom surface of the chamber 32.

The dielectrophoretic apparatus shown in FIG. 4 was produced using procedures below.
1) A pattern of electrodes was formed on an indium tin oxide (ITO) substrate by wet etching.
2) The mold of a flow channel was produced on a silicon wafer with SU-8 using lithography technology.
3) The flow channel was produced with dimethylpolysiloxane (PDMS) using the above-described mold.
4) The surfaces of the ITO substrate having the patterned electrodes and the flow channel that was produced with PDMS were activated with oxygen plasma, and the mold was attached onto the ITO substrate such that the patterned electrodes faced the flow channel.

The volume of the flow channel chamber was approximately 4 µl.

### Example 1

The above-described apparatuses were used to perform the former purification step and concentration step, and the additional purification step and concentration step using procedures below, and to collect rare cells from blood and analyze the collected rare cells.

### Former purification step and concentration step

The cell capture/staining apparatus in FIG. 2 was used to label the non-target cells (i.e., white blood cells) included in blood with magnetic particles, using procedures of steps 1 to 6 below.

Step 1: 8 mL of blood containing rare cells was introduced into the cell capture/staining apparatus, and blood was filtered through the filter and cells such as white blood cells and rare cells were captured by size selection (filtering flow rate: 1000 µL/min, flow velocity: 1.444 mm/sec).

Step 2: a staining liquid 1 containing antibodies against CD45 and CD50 and a staining liquid 2 containing a biotinylated antibody and an Alexa594 labeled antibody that recognized the antibody, and Hoechst33342 were added to the filter in the stated order and allowed to react with cells, and CD45 and CD50 expressing cells (for example, white blood cells) were biotinylated.

Step 3: a suspension containing a neutravidin labeled magnetic particle was added to the upper surface of the filter and allowed to react with the biotinylated cells, and the biotinylated cells were labeled with Neutravidin labeled magnetic particles.

Step 4: cell fixation and cell membrane permeation were performed by adding a fixation liquid containing PFA (paraformaldehyde) and a membrane permeation liquid containing Tween20 to the filter in the stated order and allowing the liquids to react with cells.

Step 5: Cytokeratin expressing cells (for example, rare cells) were labeled with FITC by adding a staining liquid 3 containing an FITC labeled anti-Cytokeratin antibody to the upper surface of the filter and allowing the liquid to react with cells.

Step 6: 600 µL of a cell suspension in the upper portion of the filter was collected by feeding a sufficient amount of a dielectrophoretic liquid to the upper surface of the filter and replacing the solution with the dielectrophoretic liquid.

The number of white blood cells (non-target cells) that were included in the collected cell suspension (600 uL) was about 5000.

### Additional purification step

The cell suspension collected in step 6 above was used to perform purification with the magnetic separation apparatus shown in FIG. 3 using the procedures of steps 7 to 11 below.

Step 7: a liquid phase constituted by the cell suspension was constructed in the Safeed™ tube by introducing 600 µL of the cell suspension that was collected in step 6 through the three-way valve (three-way stopcock valve) up to the front half of the Safeed™ tube in a gas phase state in which a liquid phase was not present and the tube was filled with air.

Step 8: a continuous gas phase was formed from the syringe pump to the interface at the end of the cell suspension by closing the introduction inlet into which the cell suspension was introduced, with the three-way valve.

Step 9: thereafter, a neodymium magnet (N40, square shape, 200×15×5 (mm), 5 mm magnetization direction) was brought close to the Safeed™ tube for 15 minutes, and was allowed to stand still at room temperature.

Step 10: air was introduced into the Safeed™ tube from the syringe pump at a flow rate of 100 µL/min, the liquid in the Safeed™ tube was moved to the rear half of the tube, and was allowed to stand still at room temperature for 15 minutes.

Step 11: all of the liquid in the Safeed™ tube was ejected from the Safeed™ tube by introducing air into the Safeed™ tube from the syringe pump at a flow rate of 100 µL/min.

### Additional concentration step

The cell suspension obtained in step 11 above was used to concentrate the suspension with the dielectrophoretic apparatus shown in FIG. 4 using the procedure of step 12 below.

Step 12: cells in the cell suspension were captured by the comb electrodes and concentrated by feeding 600 µL of the cell suspension that was obtained in step 12 at a flow rate of 20 µL/min in a state in which a dielectrophoretic force was generated by applying an alternating current with 20Vp-p, 1 MHz, and a sine wave to the comb electrodes in the dielectrophoretic apparatus filled with the dielectrophoretic liquid.

### Analysis

The dielectrophoretic apparatus that was treated up to step 12 above was imaged with a fluorescence microscope (Olympus Corporation). Hoechct33342 was detected with a fluorescent filter set 1, an FITC labeled anti-Cytokeratin antibody was detected with a fluorescent filter set 2, and an Alexa594 labeled anti-CD45 antibody and an Alexa594 labeled anti-CD50 antibody were detected with a fluorescent filter set 3.

### Fluorescent filter set

- Wavelength of the fluorescent filter set 1: EX (excitation wavelength) was 365 ± 5 nm, DM was 400 n or more, BA (fluorescence wavelength) was 470 ± 20 nm.
- Wavelength of the fluorescent filter set 2: EX (excitation wavelength) was 477.5 ± 17.5 nm, DM was 505 nm or more, BA (fluorescence wavelength) was 525 ± 10 nm.
- Wavelength of the fluorescent filter set 3: EX (excitation wavelength) was 580 ± 10 nm, DM was 600 nm or more, BA (fluorescence wavelength) was 625 ± 15 nm.

Image analysis software (product name NIS-Elements NIKON CORPORATION) was used to analyze the acquired image, and detect cells with FITC positive, Alexa594 negative, and Hoechct33342 positive cells serving as rare cells, and Alexa594 positive and Hoechct33342 positive cells serving as white blood cells.

### Collection

The cell suspension was collected from the dielectrophoretic apparatus using the procedure of step 13 below after the analysis.

Step 13: in a state in which an alternating current was not applied, that is, a dielectrophoretic force was not generated, 20 µL of a reagent mentioned in step 14 below was introduced from the outlet of the dielectrophoretic apparatus, and the cell suspension that was pushed to the inlet was collected.

### Gene detection

The cell suspension that was collected in step 13 above was subjected to EGFR genetic analysis using the procedures of steps 14 to 17 below.

Step 14: a reagent containing nonionic surfactant (NP-40) and a proteolytic enzyme (proteinase K) were mixed together and heated at 56°C for 5 minutes.

Step 15: the mixture was further heated at 95°C for 15 minutes.

Step 16: the mixture was mixed with a PCR reaction liquid containing a fluorescent probe for Tm analysis without performing nucleic acid purification.

Step 17: variants were determined through Tm analysis after 2 step-PCR constituted by 2 stages.

### PCR conditions

Condition 1: 2-step (thermal denaturation step → annealing/elongation step) PCR constituted by 2 stages

Condition 2: thermal denaturation time of 1st stage was 2 seconds, and thermal denaturation time of 2nd stage was 1 second

Condition 3: annealing/elongation temperature of 1st stage was 56°C and annealing/elongation temperature of 2nd stage was 58°C

Condition 4: the number of cycles of 1st stage was 10 and the number of cycles of 2nd stage was 50

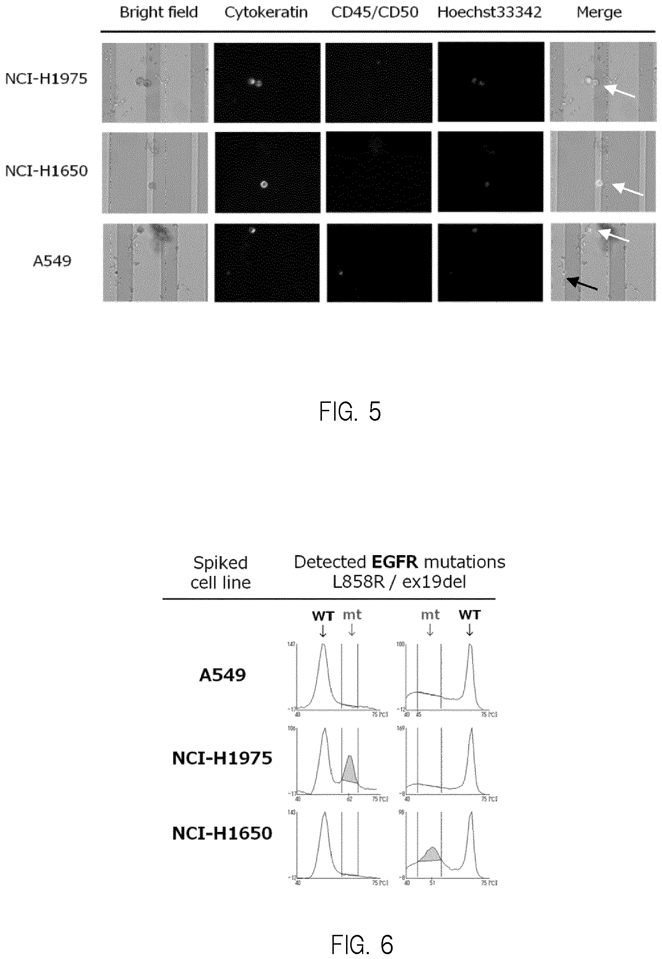

As shown in FIG. 5, rare cells (white arrow) and white blood cells (black arrow) were detected using the method of the Example.

As shown in FIG. 6, EGFR genetic mutation was detected using the method of the Example.

**Table 1**

| Spiked cell line | EGFR genotype | Expected count (cells/8mL) | Detection | | | Recovery Volume (*µ*L) |
|---|---|---|---|---|---|---|
| | | | count (cells) | Remained WBC count (cells) | EGFR genotype L858R/ex19del | |
| NCI-H1975 | L858R | 7_{(SD±3)} | 4 | 129 | L858R/Wild Type | 20 |
| NCI-H1650 | Ex19-del | 8_{(SD)±3)} | 2 | 25 | Wild Type/ex19del | 20 |
| A549 | Wild type | 6_{(SD±2)} | 4 | 199 | Wild Type/Wild Type | 20 |

As shown in Table 1, 50 µL or less of the sample having a rare cell abundance ratio of 1% or more was acquired by treating a blood sample containing 10 or fewer rare cells using the method of the Example.

### Comparative Example 1

An experiment was performed using a method similar to that of Example 1 except that the second purification step, the second concentration step, and the genetic analysis step were not performed. The results are shown in Table 2 below.

**Table 2**

| Test | Remained WBC count (cells) | Recovery Volume (*µ*L) |
|---|---|---|
| #1 | 3072 | 600 |
| #2 | 3441 | 600 |
| #3 | 5430 | 600 |

As shown in Table 2, Comparative Example 1 had a large number of white blood cells that remained and a large amount of the sample, and showed the results that 1% or more of the rare cell abundance ratio and 50 µL or less of the sample were not achieved.

### Comparative Example 2

An experiment was performed using a method similar to that of Example 1 except that the second concentration step and the genetic analysis step were not performed. The results are shown in Table 3 below.

**Table 3**

| Test | Remained WBC count (cells) | Recovery Volume (*µ*L) |
|---|---|---|
| #1 | 408 | 600 |
| #2 | 375 | 600 |
| #3 | 471 | 600 |

As shown in Table 3, Comparative Example 2 had a large amount of the sample, and showed the results that 50 µL or less of the sample was not achieved.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A method for collecting rare cells, the method comprising:
filtering a specimen comprising target rare cells and non-target cells with a filter capable of capturing the target rare cells;
labeling the non-target cells among the cells captured by the filter, at the filter
separating the labeled non-target cells from a solution comprising the rare cells and the labeled non-target cells, which solution was collected from the filter, to collect a solution comprising the rare cells; and
capturing the rare cells from the solution comprising the rare cells.

2. The collection method according to claim 1,
wherein the non-target cells are white blood cells.

3. The collection method according to claim 1 or 2,
wherein 10 ml of the specimen comprises 1 or more and 100 or fewer rare cells.

4. The collection method according to any one of claims 1 to 3,
wherein the labeling comprises magnetically labeling the non-target cells.

5. The collection method according to any one of claims 1 to 4,
wherein the separating comprises deflecting the labeled non-target cells in a direction that is different from the gravity direction, and fixing the deflected non-target cells.

6. The collection method according to any one of claims 1 to 5,
wherein the capturing comprises generating an electric field.

7. The collection method according to any one of claims 1 to 6,
wherein the rare cells are captured by dielectrophoresis.

8. The collection method according to any of claims 1 to 7, comprising:
collecting the captured rare cells with a collection liquid.

9. The collection method according to claim 8,
wherein the amount of the collection liquid is 1 pL or more and 100 µL or less.

10. The collection method according to claim 8 or 9,
wherein the ratio of the rare cells with respect to all of the cells collected with the collected liquid is 0.1% or more and 100% or more.

11. The collection method according to any of claims 1 to 10,
wherein the specimen comprises a greater amount of the non-target cells than the rare cells.

12. The collection method according to any of claims 1 to 11,
wherein the ratio of the rare cells with respect to the non-target cells in the specimen is 1% or less.

13. The collection method according to any of claims 1 to 12,
wherein the specimen is blood.

14. The collection method according to any one of claims 1 to 13,
wherein the labeling step comprises magnetically labeling the non-target cells, the separating step comprises supplying a suspension collected after the labeling step into a flow channel in which a magnetic filed is formed, deflecting, in the flow channel, the magnetically labeled non-target cells in a direction that is different from the direction in which the target cells are deflected, and the capture step comprises capturing the rare cells by dielectrophoresis.

15. The collection method according to claim 14,
wherein the magnetically labeled non-target cells are fixed to the inner wall surface of the flow channel in the deflection direction.
